# EUROPEAN PATENT SPECIFICATION

(11) **EP 4 308 696 B1**
(45) Date of publication and mention of the grant of the patent: **04.11.2026**
(21) Application number: 22716159.3
(22) Date of filing: 15.03.2022
(51) Int. Cl.: C12N 9/14, C07K 14/25, C12Q 1/34, C12Q 1/00, C12Q 1/06, C12Q 1/6869

(54) **MODIFIED SHIGELLA APYRASE AND USES THEREOF**
MODIFIZIERTE SHIGELLA-APYRASE UND VERWENDUNGEN DAVON
SHIGELLA APYRASE MODIFIÉE ET SES UTILISATIONS

(30) Priority: 16.03.2021 SE 2150297
(43) Date of publication of application: 24.01.2024
(73) Proprietor: Mitti Lifetek AB, 171 42 Solna (SE)
(72) Inventor: ASALAPURAM RAMACHANDRAN, Pavankumar, 171 42 Solna (SE); SANGITH, Nikhil, Adyar Chennai 600020 (IN)
(74) Representative: Novitas Patent AB
(86) International application number: PCT/EP2022/056739
(87) International publication number: WO 2022/194885

(56) References cited:
- WO-A1-2008/002157
- WO-A1-2016/071497
- SARLI SERENA ET AL: "Ala160 and His116 residues are involved in activity and specificity of apyrase, an ATP-hydrolysing enzyme produced by enteroinvasive Escherichia coli", MICROBIOLOGY, vol. 151, no. 9, 1 September 2005 (2005-09-01), Reading, pages 2853 - 2860, XP055932585, ISSN: 1350-0872, Retrieved from the Internet <URL:https://citeseerx.ist.psu.edu/viewdoc/download?doi=10.1.1.314.9834&rep=rep1&type=pdf> DOI: 10.1099/mic.0.28142-0
- BABU M M ET AL: "Shigella apyrase - a novel variant of bacterial acid phosphatases?", FEBS LETTERS, ELSEVIER, AMSTERDAM, NL, vol. 512, no. 1-3, 13 February 2002 (2002-02-13), pages 8 - 12, XP004341340, ISSN: 0014-5793, DOI: 10.1016/S0014-5793(02)02287-1
- ANONYMOUS: "UPI000973079A", 28 January 2017 (2017-01-28), XP055932496, Retrieved from the Internet <URL:https://www.uniprot.org/uniparc/UPI000973079A> [retrieved on 20220617]

## Description

### TECHNICAL FIELD

The present invention relates to field of apyrases, in particular for the degradation of organic phosphates.

The present invention also relates to analytical and diagnostic methods where contaminating nucleotides are an issue. In particular, the present invention relates to determining or quantifying the amount of ATP present in a sample that may contain contaminating nucleotides, as well as reagents for use in such methods and production of such reagents.

### BACKGROUND TO THE INVENTION

Adenosine triphosphate (ATP) is a molecule present in all living cells. Since the concentration of ATP is fairly constant in the cell, measurement of ATP content in a sample can be used as a proxy to determine the number of viable cells. Sensitive bioluminescent assays for measuring ATP based on luciferase/luciferin are known, see e.g., US3745090. Luciferase (e.g., from firefly) is a euglobulin protein that catalyses the oxidative decarboxylation of luciferin using ATP and molecular oxygen to yield oxyluciferin, a highly unstable, single-stage excited compound that emits light upon relaxation to its ground state. This reaction emits light proportional to ATP concentration in the reaction mixture, and by measuring the intensity of the emitted light it is possible to continuously monitor the concentration of ATP present.

In many cases, samples to be analyzed for ATP content contain contaminating ATP either extracellularly or present in a contaminating type of cells. For instance, if the number of bacteria is to be quantitated in any clinical or biological sample, any ATP contained in host cells present in the sample will interfere with the measurement. In many applications, the contaminating cells may be selectively lysed, and the ATP released, resulting in that all the contaminating ATP is in extracellular form, see e. g. US4303752 or US20110076706. In other instances, ATP analogues different from ATP may be present in a sample and interfere with ATP measurement.

The contaminating extracellular ATP, otherwise unwanted ATP or ATP analogues can be reduced or eliminated by hydrolyzing them with an enzyme called apyrase (ATP-diphosphohydrolase, E-type ATPase, ATPDase, NTDase EC 3.6.1.5). Apyrase is frequently used in methods for determining bacterial ATP in the presence of mammalian cells, where the mammalian cells are first selectively lysed, and apyrase used to degrade extracellular ATP leaving the bacterial ATP unaffected. After completion of the reaction, the apyrase can be inactivated, and the intracellular ATP of bacterial cells is released to measure bacterial ATP by the addition of luciferin/luciferase. Light emission is measured before and after the addition of a known amount of ATP standard, as internal control. The bacterial ATP (in moles) is calculated by multiplying the ratio of the light before and after adding the ATP standard with the amount of added standard. Typically, bacterial cells contain around 1 attomole of ATP per cell, making it possible to estimate the number of bacterial cells from the amount of ATP detected. It is an object of the present invention to provide improvements for such analytical and diagnostic methods.

In this context, the most commonly used apyrase is *Solanum tuberosum* apyrase (STA). STA exists in several isoforms and each isoform differs in ATP-degradation activity. However, the efficiency of STA in the above methods is limited by the accumulation of ADP and uncharacterized ATP-analogues in the degradation reaction when using STA. Accumulation of such contaminants inhibit the ATP degradation capability allowing some of the contaminating ATP to remain intact, which in turn limits the sensitivity of the ATP determination assays and increases the background signal. For a discussion on the limitations of STA, see WO199402816.

Further, most embodiments of the DNA sequencing method pyrosequencing (see e.g., international patent applications PCT/GB1997/002631 and PCT/GB1997/003518, or US patent applications US2013/0045876 and US2013/0189717) also rely on quantitation of ATP, usually by the luciferase/luciferin assay. At the end of every sequencing cycle, all the unincorporated nucleotides and excess ATP must be eliminated by e.g., apyrase. As detailed above, the apyrases presently used in DNA sequencing applications have problems in achieving complete degradation due to the quality of enzyme (contamination of NDP kinase), substrate specificity and batch-to-batch variations that not only results in *drop off* and non-linear peaks but also affects DNA sequencing of long strands.

The use of wild-type *Shigella flexneri* apyrase in degrading contaminating nucleoside diphosphates and/or triphosphates has been disclosed in WO 2016/071497 A1.

It is an object of the present invention to provide an alternative or improved apyrase for the applications discussed above as well as other applications.

### DEFINITIONS

The following terms and words have the meanings as defined below in the context of the present disclosure.

*Shigella flexneri* apyrase (SFA) is defined as apyrase derived from *Shigella flexneri,* at any suitable degree of purity. The SFA may be produced by non-recombinant means or by recombinant DNA technology. Recombinant *Shigella flexneri* apyrase is abbreviated rSFA herein. The native SFA has the sequence according to SEQ ID NO: 1. Apyrase activity refers to capacity to catalyse hydrolysis of nucleoside triphosphates to nucleoside diphosphates, and hydrolysis of nucleoside diphosphates to nucleoside monophosphates.

*Sequence identity* expressed in percentage is defined as the value determined by comparing two optimally aligned sequences over a comparison window, wherein a portion of the sequence in the comparison window may comprise additions or deletions (i.e., gaps) as compared to the reference sequence (which does not comprise additions or deletions) for optimal alignment of the two sequences. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Unless indicated otherwise, the comparison window is the entire length of the sequence being referred to. In this context, optimal alignment is the alignment produced by the BLASTP algorithm as implemented online by the US National Center for Biotechnology Information (see The NCBI Handbook [Internet], Chapter 16), with the following input parameters: Word length=3, Matrix=BLOSUM62, Gap cost=11, Gap extension cost=1.

*ATP analogues* refer to compounds with structural and functional similarity to ATP which compete with ATP for binding to enzymes that specifically interact with ATP. In the present context, the term only applies to compounds that are substrates for SFA as defined above. Preferably, the term refers to compounds that can substitute ATP as substrate for luciferases, such as firefly luciferase. In preferable embodiments, the term includes nucleoside diphosphates and nucleoside triphosphates. In more preferable embodiments, the term refers to adenosine diphosphate, deoxyadenosine alpha-thio triphosphate, adenosine tetra-phosphate, deoxyadenosine triphosphate, deoxyadenosine diphosphate, guanosine triphosphate, guanosine diphosphate, deoxyguanosine triphosphate, deoxyguanosine diphosphate, thymidine triphosphate, deoxythymidine triphosphate, thymidine diphosphate, deoxythymidine triphosphate, cytidine diphosphate, deoxycytidine triphosphate, cytidine triphosphate, deoxycytidine diphosphate, uridine diphosphate and uridine triphosphate. In most preferable embodiments, ATP analogues refers to: adenosine diphosphate, deoxyadenosine alpha-thio triphosphate, adenosine tetra-phosphate, deoxyadenosine triphosphate, deoxyadenosine diphosphate, guanosine triphosphate, guanosine diphosphate, deoxyguanosine triphosphate and deoxyguanosine diphosphate.

### BRIEF DESCRIPTION OF THE FIGURES

**Figure 1** is an SDS-PAGE analysis showing efficient induction of the mutant apyrase (SEQ ID NO: 1).
**Figure 2** is a crude colorimetric activity assay showing ATP degradation activity in periplasmic samples.
**Figure 3** shows the successful purification of the mutant apyrase (SEQ ID NO: 1).
**Figure 4** is a graph demonstrating indistinguishable ATP degradation activity of the wild-type (SEQ ID NO: 1) and mutant apyrase (comprising SEQ ID NO:3).
**Figure 5** demonstrates that the wild-type (SEQ ID NO: 1) is not active in degrading an organic phosphate pNPP while the mutant apyrase (comprising SEQ ID NO:3) shows considerable activity.
**Figure 6** further demonstrates that the mutant apyrase (comprising SEQ ID NO:3) shows much improved activity on the organic phosphate pNPP. Alkaline phosphatase is also shown for comparison.
**Figure 7****:** SDS-PAGE analysis of mutant proteins purified by affinity chromatography.
**Figure 8** shows A) hydrolysis of pNPP by ApiTwo mutants and B) hydrolysis of TPP by ApiTwo mutants.
**Figure 9** illustrates identification of the site crucial for pNPP and TPP hydrolysis. A) Model of ApiOne denoting the active site and crucial loop regions (magenta). Mutated residues are denoted in red. B) Effect of double mutant on pNPP hydrolysis. C) Effect of double mutant on TPP hydrolysis.
**Figure 10** illustrates the role of the loop in recruiting substrates and effect of temperature on hydrolysis.

### SUMMARY OF THE INVENTION

The scope of the invention is defined in the appended set of claims.

### DETAILED DESCRIPTION

The present invention provides a mutated variant of the *Shigella flexneri* apyrase (SFA, Example 1), which has similar activity towards ATP degradation (dephosphorylation) as the wild type (wt) apyrase (Example 2). However, the mutated variant exhibits considerably higher affinity and catalytic activity towards dephosphorylation of certain organic phosphates compared to the wild-type Shigella apyrase (Example 3). Experiments shown in Examples 4 and 5 further pinpoint the key mutations behind the improved activity and characterize the mutated enzyme.

The value of the Michaelis constant Kₘ is numerically equal to the [substrate] at which the reaction rate is at half-maximum (1/2 x Vₘₐₓ), and is an inverse measure of the substrate's affinity for the enzyme-as a small Kₘ indicates high affinity, meaning that the rate will approach the maximum rate (Vₘₐₓ) with lower [substrate] than reactions with a higher value of Kₘ. The Kₘ constant is independent of the purity or concentration of the enzyme but varies between substrates and reaction conditions.

### Mutated apyrase with broadened substrate specificity

Thus, in a first aspect, the present invention provides an apyrase enzyme, characterized by that:
a. the apyrase comprises a polypeptide sequence having at least 90% sequence identity to the wild-type Shigella flexneri apyrase of SEQ ID NO:1,
   wherein said sequence differs from SEQ ID NO:1 at least in that the sequence comprises at least one amino-acid substitution of a residue aligning with a residue selected from: F53, L66 and E77;
   and
b. the apyrase catalyzes the dephosphorylation of at least one organic phosphate with at least 10-fold lower Kₘ compared to the apyrase of SEQ ID NO:1.

The apyrase may catalyze the dephosphorylation of the at least one organic phosphate with at least 50-fold lower, preferably at least 100-fold lower Km compared to the apyrase of SEQ ID NO:1.

The at least one organic phosphate may refer to para-Nitrophenylphosphate (pNPP). Alternatively, the at least one organic phosphate may refer to thiamine pyrophosphate (TPP). Preferably, the at least one organic phosphate refers to both pNPP and TPP.

The apyrase may exhibit a Kₘ of less than 30 mM for dephosphorylation of para-Nitrophenylphosphate (pNPP), more preferably less than 10 mM, even more preferably less than 2 mM, most preferably less than 1 mM. The apyrase Kₘ for dephosphorylation of pNPP is preferably measured at pH7.5 and at a temperature of 37°C, preferably in a 50 mM Tris buffer at pH7.5.

The apyrase may exhibit a Kₘ of less than 30 mM for dephosphorylation of thiamine pyrophosphate (TPP), more preferably less than 20 mM, even more preferably less than 10 mM, most preferably less than 6 mM. The apyrase Kₘ for dephosphorylation of TPP is preferably measured at pH7.5 and at a temperature of 37°C, preferably in a 50 mM Tris buffer at pH7.5.

Preferably, the apyrase of the first aspect is capable of catalyzing the dephosphorylation of ATP. More preferably, the apyrase catalyses the dephosphorylation of ATP with a Kₘ differing less than 5-fold from that of the apyrase of SEQ ID NO:1, preferably less than 2-fold. The apyrase may exhibit a Kₘ of less than 10 mM for the dephosphorylation of ATP, preferably less than 5 mM. The apyrase Kₘ for the dephosphorylation of ATP may be measured at pH7.5 and at a temperature of 37°C, preferably in a 50 mM Tris buffer at pH7.5.

The substitutions preferably comprise a substitution of a residue aligning with F53. More preferably, the substitutions comprise the substitution F53V.

The substitutions preferably comprise a substitution of a residue aligning with L66. More preferably, the substitutions comprise the substitution L66V.

The substitutions preferably comprise a substitution of a residue aligning with E77. More preferably, the substitutions comprise the substitution E77V.

Preferably, the sequence does not comprise the substitution G63R, more preferably any substitution G63. Preferably, the sequence does not comprise the substitution of S97P, preferably any substitution of S97. Preferably, the sequence does not comprise the substitution H116L, preferably any substitution of H166. More preferably, the sequence does not comprise any substitution of any of G63, S97 or H116. Most preferably, the sequence does not comprise any of the substitutions G63R, S97P and H116L.

The apyrase of the first aspects preferably comprises a polypeptide sequence having at least 95% sequence identity to SEQ ID NO: 1.

More preferably, the apyrase comprises a polypeptide sequence according to SEQ ID NO: 3. Most preferably, the apyrase consists of the polypeptide sequence according to SEQ ID NO: 3.

### Method involving reducing the amount of contaminating nucleoside diphosphates and nucleoside triphosphates

The International Patent Application WO2016/071497 discloses methods utilizing a wild-type *Shigella flexneri* apyrase in reducing the amount of contaminating or unwanted nucleoside diphosphates and/or nucleoside triphosphates in several applications, including ATP determination and sequencing-by synthesis. The mutant enzymes of the first aspect described herein are useful in these methods.

In a second aspect, the present invention relates to a method for reducing the amount of contaminating nucleoside diphosphates and/or nucleoside triphosphates, comprising the steps of
a. providing a sample containing contaminating nucleoside diphosphates and/or nucleoside triphosphates, such as ATP and/or ATP analogues including deoxyribonucleoside triphosphates;
b. reducing the amount of the contaminating nucleoside diphosphates and/or nucleoside triphosphates in the sample with an apyrase enzyme, wherein said apyrase enzyme an apyrase according to the first aspect; and
c. performing an analysis of the sample, wherein said analysis comprises an assay that would have been affected by the contaminating nucleoside diphosphates and/or nucleoside triphosphates had they not been reduced in step b.

In a third aspect, the present invention relates to a method for determining the amount of ATP in a sample, comprising the steps of:
a. providing a sample containing contaminating ATP and/or ATP analogues including deoxyribonucleoside triphosphates;
b. reducing the amount of contaminating ATP and/or ATP analogues in the sample by degradation with an apyrase enzyme;
c. making the ATP to be determined available for determination; and
d. determining the amount of ATP to be determined in the sample,
wherein the apyrase enzyme is an apyrase according to the first aspect.

In a fourth aspect, the present invention relates to a method for determining the amount of ATP present in a first population of cells in a sample, comprising the steps of:
a. providing a sample containing contaminating ATP and/or ATP analogues including deoxyribonucleoside triphosphates;
b. reducing the amount of contaminating ATP and/or ATP analogues in the sample by degradation with an apyrase enzyme;
c. liberating the ATP to be determined from the first population of cells; and
d. determining the amount of liberated ATP; wherein the apyrase enzyme in step (b) is an apyrase according to the first aspect.

In a preferred embodiment, the liberation step (b) involves lysis of the first population of cells; the first population of cells comprises bacterial cells; and the sample is a biological sample from an animal or a human.

The sample may be a blood sample, a plasma sample, a serum sample, a urine sample, a fecal sample, or a swab from a patient.

At least a fraction of the contaminating ATP may be present in a second population of cells, in which case the reduction step (a) may be preceded by a step of selective liberation of ATP from the second population of cells, wherein the second population of cells are host cells from an animal from which the sample is derived.

In a fifth aspect, the present invention relates to a method for doing pyrosequencing comprising the steps of:
a. performing a pyrosequencing reaction comprising addition of a nucleoside triphosphate;
b. converting the pyrophosphate released in step (a) into ATP via an enzymatic reaction;
c. determining the amount of ATP formed in step (b);
d. degrading unincorporated nucleoside triphosphate from step (a) and ATP formed in step (b) with an apyrase enzyme;
e. repeating the steps a-d at least once;
wherein the apyrase enzyme is an apyrase according to the first aspect.

In a sixth aspect, the present invention relates to a use of an apyrase according to the first aspect for degrading contaminating nucleoside triphosphates or nucleoside diphosphates in an analytical method.

### Dephosphorylation of organic phosphates

The mutated apyrase of the first aspect has improved activity for certain substrates such as pNPP and/or TPP. In a seventh aspect, the present invention provided a use of an apyrase according to the first aspect for dephosphorylating an organic phosphate. The seventh aspect also encompasses a method for catalyzing the dephosphorylation of an organic phosphate, comprising contacting the organic phosphate with the apyrase of the first aspect. The contacting is preferably done in an aqueous solution at a temperature of 4-50°C, more preferably 20-40°C, more preferably 36-38°C. The aqueous solution preferably has a pH of about 5.5-9.5, more preferably 6.5-8.5, yet more preferably about 7.0-8.0, most preferably about 7.5.

### General statements relating to the present disclosure

The term "comprising" is to be interpreted as including, but not being limited to.

### EXAMPLES

The following examples are not to be regarded as limiting.

### Example 1: Production of mutated Shigella apyrase

### Random mutagenesis

In order to improve the activity of apyrase and its tolerance to high salt conditions, the inventors mutated the apyrase gene randomly using an error prone PCR method starting from the wild-type *Shigella flexneri* apyrase DNA sequence (SEQ ID NO: 4) as template. The method incorporates repeated copying of the template at high MgCl₂ concentration (5 mM) with *Taq* polymerase. After every fourth cycle, one fifth of the reaction mixture was transferred to fresh PCR tube and the same process was repeated 22 times to obtain a product that will have randomly mutated nucleotides which can be identified by sequencing. The mutated gene was cloned in pET21a vector. The mutated DNA sequence obtained is presented as SEQ ID NO: 2, containing a total of 31 mutations leading to amino-acid changes (see SEQ ID NO:3 compared to the wild-type amino-acid sequence of SEQ ID NO: 1).

The protein can be divided into 4 regions where mutations have occurred
51-80: S50N, F54V, G63R, L66V, N70Q, E77V, A79I
90-128: D90T, S97T, L108M, Q113N, V120R, K124R, Y127Q
135-185: P143R, I151L, F162N, A167G, N174Q, K177R, L182F, G184A
186-246: W199Y, G206R, V213A, N219K, F223W, L227S, F234Y, T239Q, E243D

### Expression of mutated apyrase

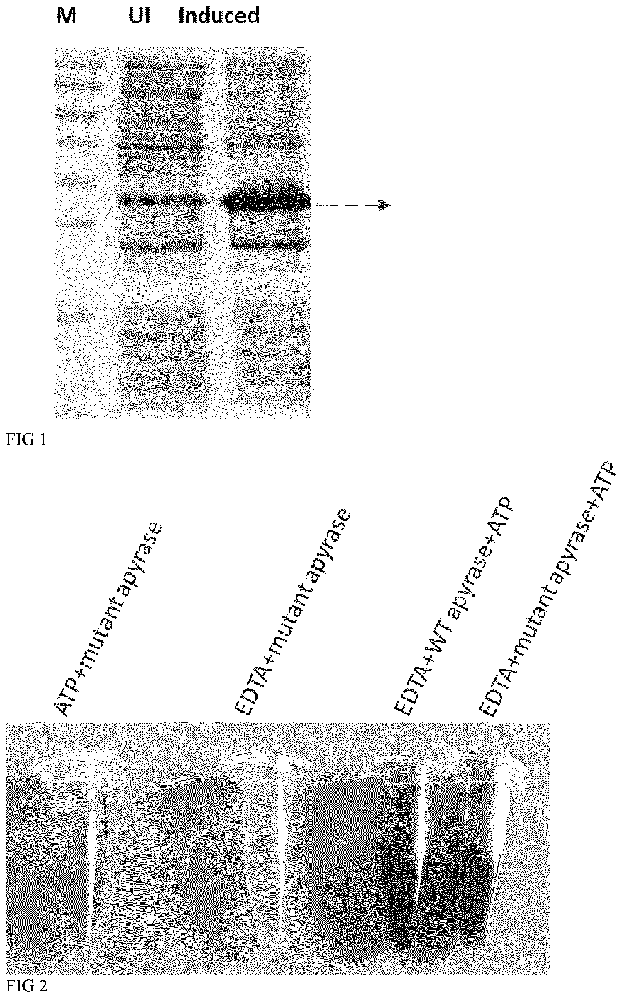

BL21(DE3) RP codon plus cells transformed with the pET21a-mutant apyrase (SEQ ID NO: 2 as coding sequence) were grown at 37°C until OD600nm reaches 0.5-0.6. Expression was induced by adding 0.6 mM IPTG and incubating the culture at 18°C for 20 hours. Cells were harvested and sonicated for checking induction. Uninduced lysate was taken as control. The induced and uninduced cells were analysed with SDS-PAGE showing effective induction (**Fig 1**, arrow).

### Whole cell activity assays

As a crude assay for confirming expression of active enzyme, a whole cell ATP degradation activity assay was performed. 100 mM ATP was prepared in 50 mM Tris, pH 7.5. After dissolution, the pH was adjusted to 7.5 using NaOH. Pellets from 1 mL culture of induced cells were taken and washed twice with saline containing 1mM Calcium chloride. The cells were treated with Lysozyme and EDTA to lyse the cell wall to release the periplasmic contents.

150 µL of 40 mM EDTA and 150 µL of 10 mM ATP was added to the pellet and mixed well, followed by incubation at 37°C for 30 minutes. Thereafter, 700 µL of AMFAS reagent (equal volumes of Solution A 5% (w/v) ammonium molybdate in 5 N H₂SO₄ and B) 1% (w/v) ferrous ammonium sulfate in double-distilled water) was added. From the results (**Fig 2**) it could be concluded that functional mutant apyrase was expressed in periplasmic fraction by this method, and it can hydrolyse ATP similar to the WT apyrase.

### Purification of His-mutant apyrase by affinity chromatography

Pellet of 500 mL culture was resuspended in 15 mL binding buffer (50 mM Tris, pH 7.5, 150mM NaCl and 20 mM imidazole). The suspension was subjected to ultrasonication on ice (Amplitude 38%, Pulse on- 1 sec, Pulse off- 1 sec for 1 minute. The sonication was repeated 6 times). The sonicated suspension was centrifuged at 14, 000 rpm for 20 minutes at 4°C.

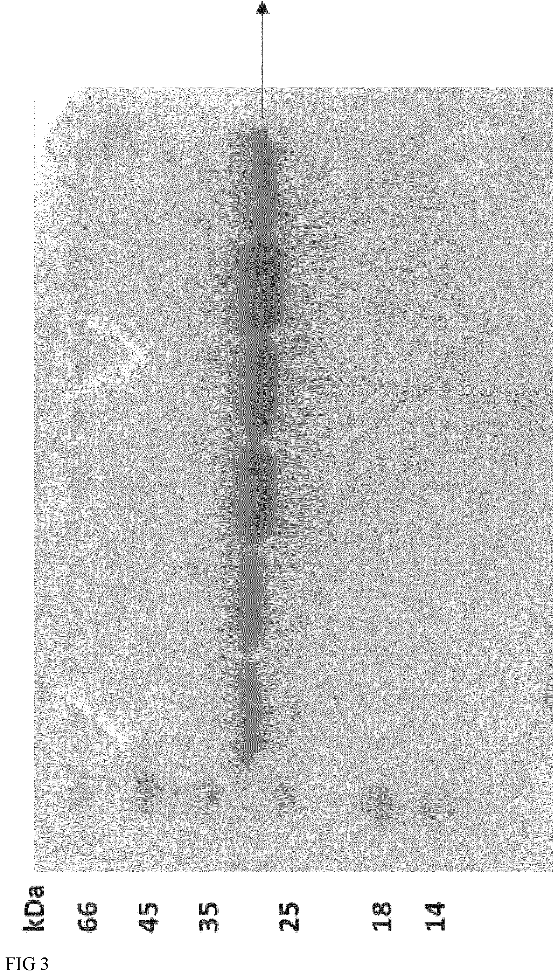

To prepare the resin, 1.0 mL of Ni- sepharose (GE Healthcare) resin was washed with 15 mL water and then with 15 mL binding buffer. The sonicated lysate and the washed resin were mixed and incubated for 1 hour at room temperature on a rocker. After this, the lysate was drained out on a column and the beads were washed thoroughly with 200 mL binding buffer. The protein was then eluted in 1.0 mL fractions using the elution buffer (50 mM Tris, pH 7.5, 10% glycerol and 250 mM imidazole). Fractions were loaded on 12% SDS PAGE to confirm the purity of the protein (**Fig 3**).

Purified protein was quantitated by Bradford method. The yield of purified apyrase from 500 mL culture was 6.3 mg.

### Example 2: Unaltered ATP hydrolysis using purified recombinant mutant apyrase

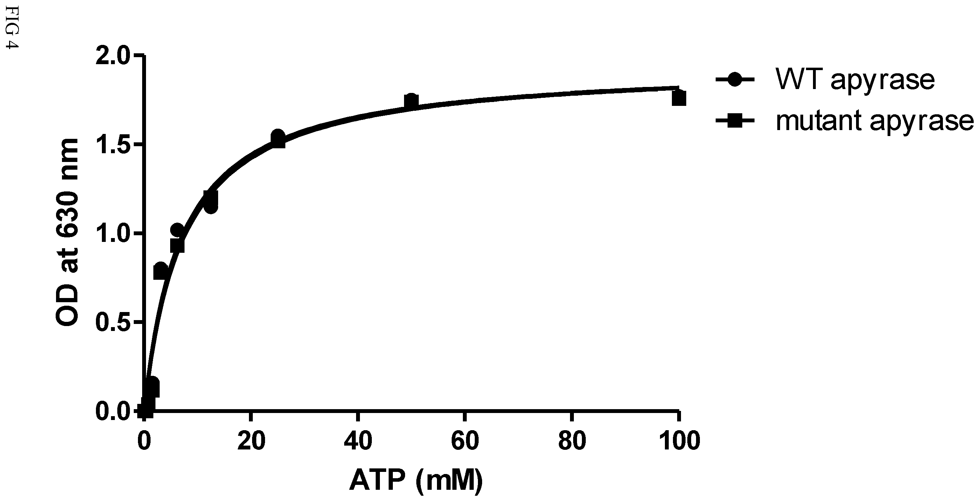

A titrated series of ATP solutions from 100 mM to 0.01 mM in 50 mM Tris, pH 7.5 were prepared and incubated with 700 ng of Apyrase (mutant prepared in Example 1 and wild-type *Shigella flexneri* apyrase as control) for 30 minutes at 37°C (100 µL reaction). After the incubation, 100 µL AMFAS reagent was added and OD was taken at 630 nm using multimode reader (Biotek instruments). The color formation is proportional to the amount of ATP degraded (dephosphorylated to ADP or AMP). As seen in **Fig 4****,** the activity mutant apyrase with respect to ATP degradation was indistinguishable from the wild-type enzyme. The calculated Kₘ of the wild type enzyme was 3.3 mM and for the mutant 3.6 mM. In conclusion, there was no significant difference in affinity for ATP between the mutant and the wild-type apyrases. Specific activity at 10 mM ATP was determined as 6.3 µmoles/min/mg for the mutant apyrase.

### Example 3: Enhanced activity on organic phosphates (pNPP- para nitrophenol phosphate)

Wild type apyrase does not hydrolyse many organic phosphates such as pNPP (Bhargava et al. Current Science 1995 vol 68:3 293-300). To test whether the mutant hydrolyses this substrate a pNPP dephosphorylation assay was performed. The reaction can be described as follows:
Substrate: para Nitro phenol phosphate (pNPP) 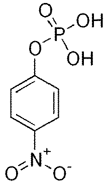
Products: para Nitro phenol (PNP, yellow colour, absorbance at 405 nm) and phosphate
pNPP + Apyrase→ PNP + Pᵢ

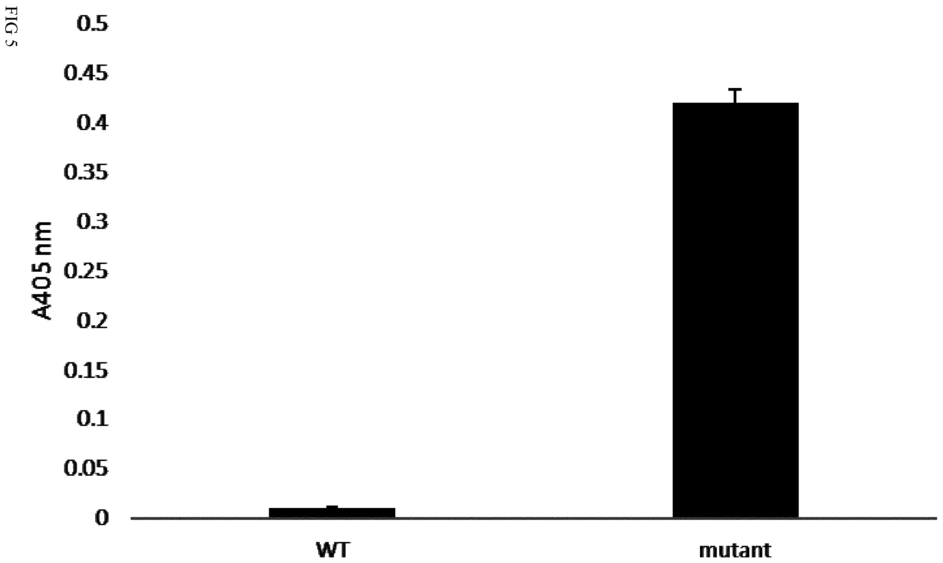

Wild-type and mutant apyrase from Example 1 (2 µg each) were incubated with 3 mM pNPP (pNPP was dissolved in 50 mM Tris pH 7.5) in a 100 µL reaction mixture. Control reaction with pNPP lacking WT and mutant apyrase was also included. These were incubated at 37°C for 15 minutes and absorbance was taken at 405 nm. It is clear from this assay (**Fig 5**) that the mutant has acquired the ability to hydrolyse pNPP, while wildtype enzyme lacks such activity.

To further study the capability of the mutant enzyme to hydrolyse pNPP, the substrate (pNPP) was titrated and incubated with the mutant apyrase. As controls, alkaline phosphatase (known to hydrolyse pNPP) and the wildtype enzyme were also included.

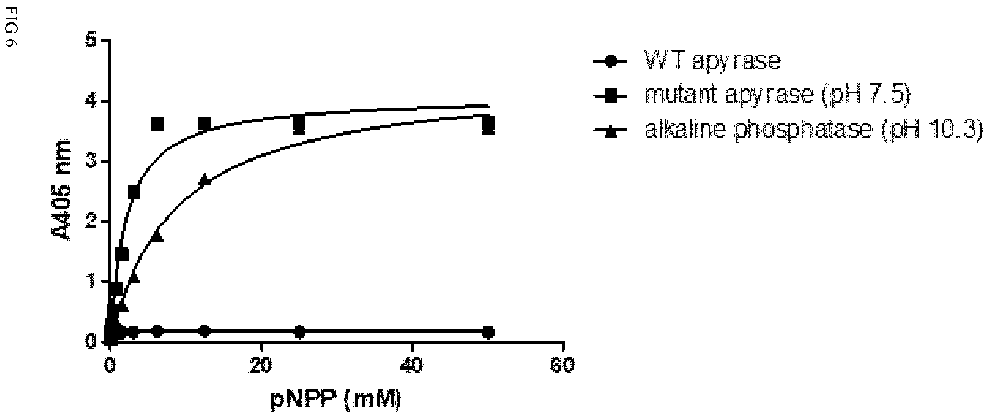

The enzymes (2 µg) were incubated with different concentrations of pNPP (dissolved in 50 mM Tris, pH 7.5) in a 100 µL reaction mixture. For alkaline phosphatase, pNPP was dissolved in carbonate buffer (pH 10.3). Control reaction with pNPP lacking WT, mutant apyrase and alkaline phosphatase was also included. These were incubated at 37°C for 15 minutes and absorbance was taken at 405 nm. The results are shown in **Fig 6** and **Table 1** below.

**Table 1: Kₘ values for pNPP**

| **Enzyme** | **Kₘ for pNPP (mM)** |
|---|---|
| Wild-type apyrase | Too low affinity to determine |
| Mutant apyrase | 2.1 |
| Alkaline phosphatase | 8.4 |

In conclusion, the mutant apyrase of Example 1 is very potent in catalysing the hydrolysis pNPP while the wild-type apyrase does not have appreciable activity. It can be concluded that the mutations resulted in significantly broadened substrate selectivity, and that the mutant apyrase would be useful in applications requiring the dephosphorylation of organic phosphates.

### Example 4: Screening of effective point mutations for broadened substrate specificity

A screening effort was undertaken to find out which of the mutations generated in the random mutagenesis experiments (Example 1) were behind the observed broadening of substrate specificity (while retaining activity on ATP).

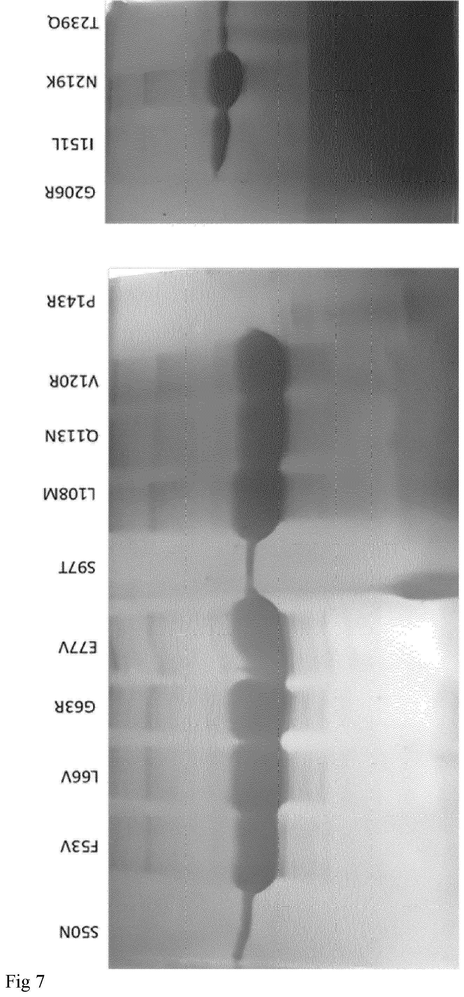

All the mutations from the random mutagenesis were individually introduced into the *Shilegella flexneri* apyrase sequence (SEQ ID NO: 1, termed ApiOne herein) by site directed mutagenesis by PCR using specific primers. However, not all reactions successfully generated PCR products by agarose gel electrophoresis. The successful mutations were transformed into the expression host and proteins were expressed and purified to homogeneity by affinity chromatography. Again, some mutations resulted in poor protein levels as shown by SDS-PAGE (Figure 7).

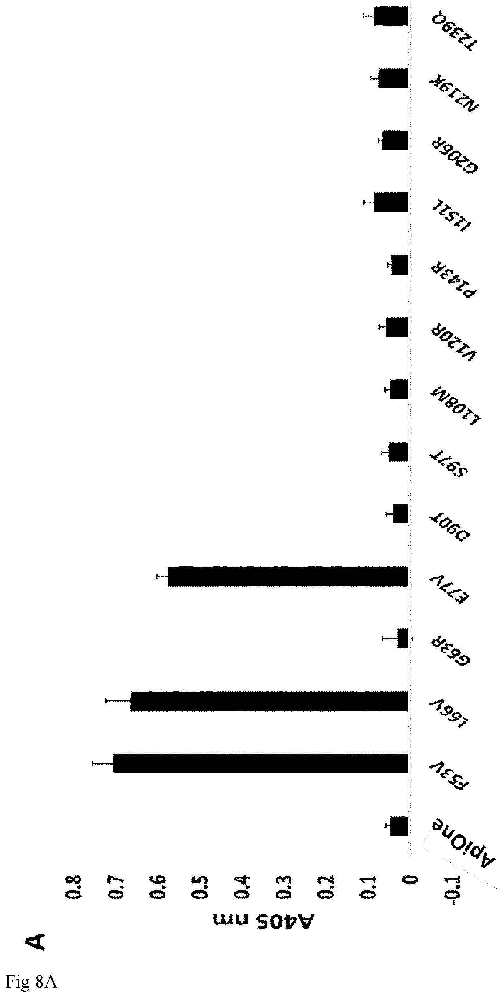

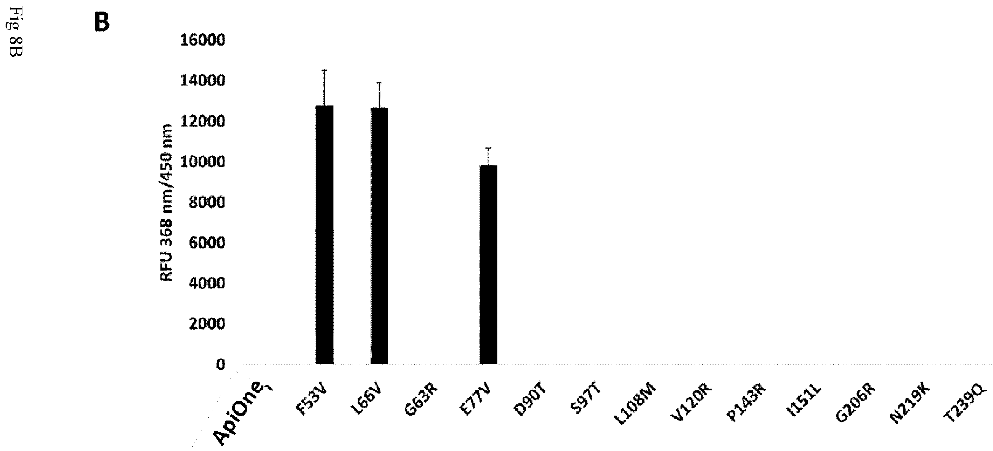

We screened whether the mutants could hydrolyse organic phosphates. Of the all the mutants, only 3 mutants (F53V, L66V and E77V, termed ApiTwo herein) could hydrolyse pNPP and TPP. Other substrates like 2-Glycerophosphate, Pyridoxal phosphate and phenyl phosphate were not hydrolysed by the mutants (Table 2 and Figure 8A,B).

**Table 2. Organic substrates screened for hydrolysis by generated single-substitution mutants.**

| **Substrate** | **Hydrolysis** |
|---|---|
| para-Nitro phenyl phosphate (pNPP) | Yes |
| 2-glycerophosphate | No |
| Thiamine pyrophosphate (TPP) | Yes |
| Phenyl phosphate | No |
| Pyridoxal phosphate | No |

As seen from Figure 8, only the mutations F53V, L66V and E77V each resulted in the broadened substrate specificity (at roughly similar level of activity). The change was especially striking for TPP, for which the wild-type enzyme has no detectable activity.

### Structure-function analysis

The mutations F53V, L66V and E77V are outside the catalytic site proposed by Babu et. *al* (Babu, M.M., Kamalakkannan, S., Subrahmanyam, Y.V., Sankaran, K., 2002. Shigella apyrase- -a novel variant of bacterial acid phosphatases? FEBS letters 512(1-3), 8-12).

Therefore, it was not surprising to see that these substrates hydrolysed ATP with the same affinity as that of the wild-type enzyme (Table 3). However, it was necessary to understand the structural basis of recognition of both pNPP and TPP, whose hydrolysis resulted due to mutations close to the loop regions outside the pocket. The role of the loop regions (AYYENFG and TPDKDEKMAIT) in substrate binding in acid phosphatases is very well known. Their conformational changes during binding play a key role in hydrolysis (Babu et. *al,* 2002).

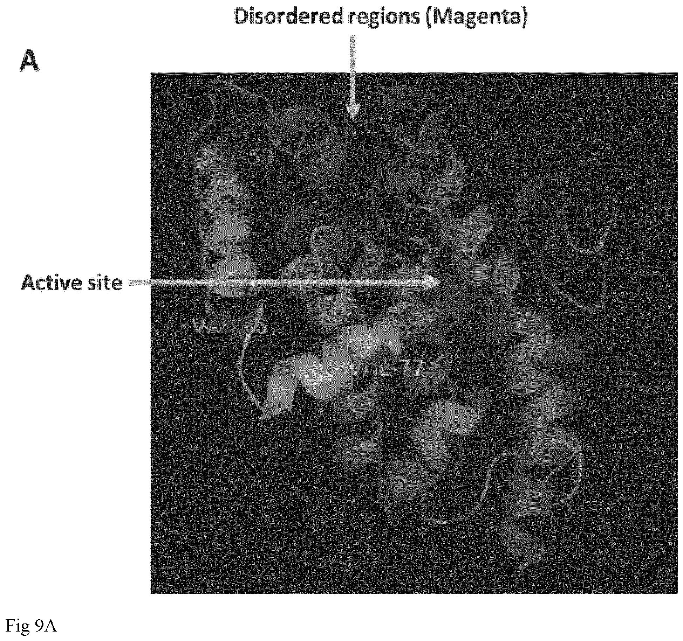

Computational modelling of ApiOne based on the template structure of acid phosphatase from *Escherichia blattae* clearly showed that the regions AYYENFG and TPDKDEKMAIT are in the loops (Figure 9A). Therefore, the new mutations have enabled conformational changes in the loop regions enough to induce hydrolysis of pNPP and TPP.

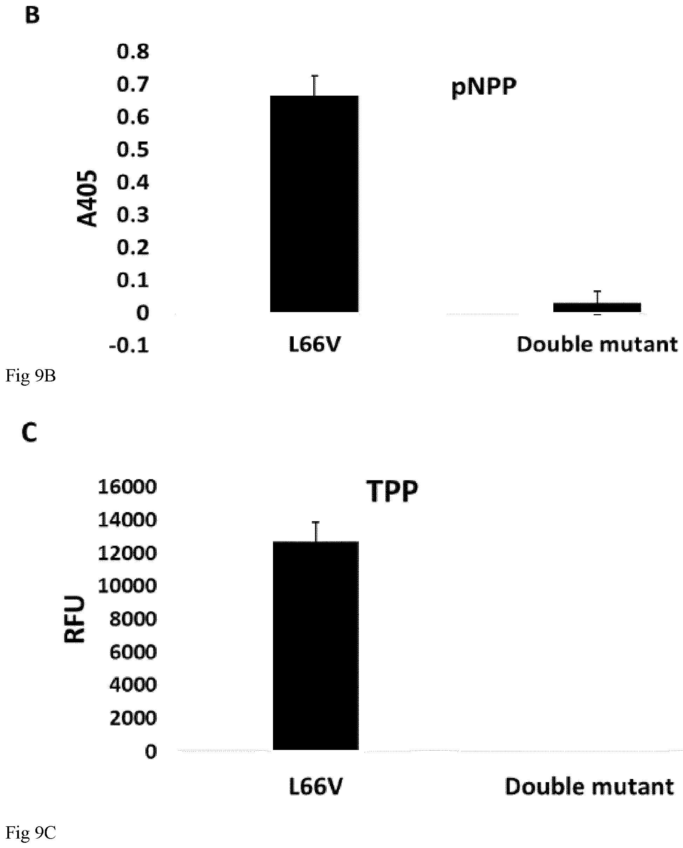

When we mutated glycine 63 residue to arginine (G63R) in the ApiTwo mutant L66V (referred as double mutant from now on), it completely inhibited the ability to hydrolyse pNPP and TPP (Figure 9B and 9C) while retaining the ATP-hydrolysing activity of the wild-type enzyme (termed herein: ApiOne). This clearly proves that this region is crucial for interaction of pNPP and TPP and their subsequent hydrolysis. These results clearly show that the mechanisms for hydrolysis of ATP/ADP and pNPP/TPP, respectively are entirely different.

**Table 3. Affinity of ApiOne, ApiTwo mutant L66V and the Double mutant to different substrates.**

| **Protein** | **Substrate** | **Activity** | **Affinity (Kₘ)** |
|---|---|---|---|
| ApiOne | ATP | Y | 20.0 µM |
| L66V | | Y | 18.5 µM |
| Double mutant | | Y | 20.7 µM |
| ApiOne | pNPP | N | - |
| L66V | | Y | 0.76 mM |
| Double mutant | | N | - |
| ApiOne | TPP | N | - |
| L66V | | Y | 5.6 mM |
| Double mutant | | N | - |

### Example 5: Thermal stability experiments reiterate the dual mechanism of catalysis in ApiTwo.

ApiTwo mutants were incubated at 25°C, 55°C and 70°C and their ability to hydrolyse different substrates were tested. ApiTwo mutants still retained significant activity (60-70%) for ATP/ADP hydrolysis. However, heating at 55°C led to significant abrogation of pNPP and TPP hydrolysis (8-10% activity) (Table 4). Cooling the proteins to 25°C enabled almost 85-95% activity in the case of ATP hydrolysis, but the recovery for pNPP and TPP hydrolysis was extremely poor (20-30% activity) (Table 5). This further reinstates the role of mutated regions (which are close to the crucial loop regions) and the differential mechanism of hydrolysis in ApiTwo mutants.

**Table 4. Activity of ApiTwo mutants after 2 h of incubation (in relative %)**

| **Substrate** | **25°C** | **55°C** | **70°C** |
|---|---|---|---|
| **ATP** | 100 | 83 | 63 |
| **ADP** | 100 | 82 | 68 |
| **pNPP** | 100 | 10 | 8 |
| **TPP** | 100 | 11 | 10 |

**Table 5. Activity of apyrase mutants after 1 h of cooling (in relative %)**

| **Substrate** | **55°C to 25°C** | **70°C to 25°C** |
|---|---|---|
| **ATP** | 96 | 86 |
| **ADP** | 97 | 88 |
| **pNPP** | 30 | 21 |
| **TPP** | 32 | 27 |

### ApiTwo- An enzyme with both pyrophosphatase and acid phosphatase activity

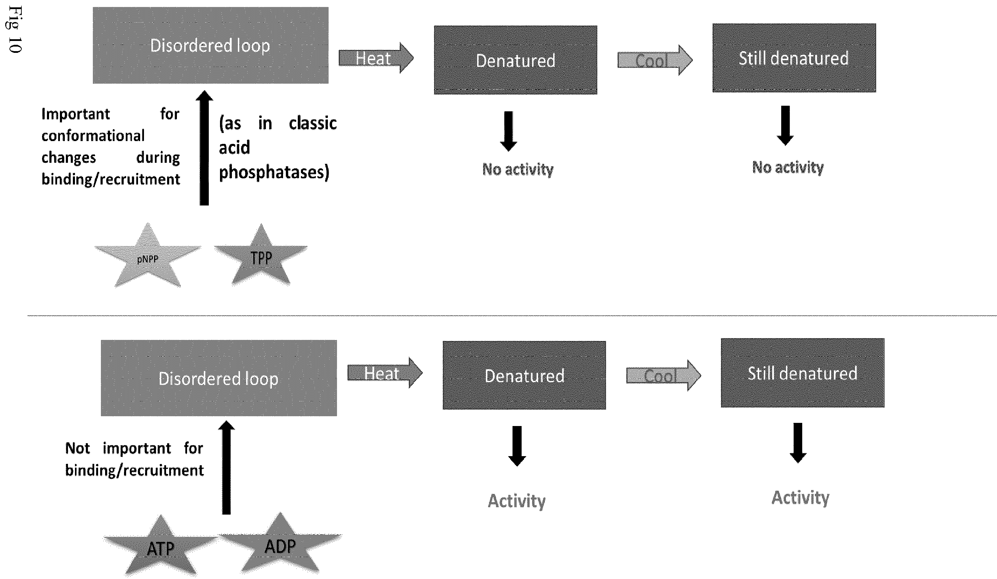

It is well known there is high degree of homology between the protein sequences of ApiOne and acid phosphatase of *Escherichia blattae.* The movement of the loop regions are important during substrate binding. A pyrophosphatase like apyrase generally does not hydrolyse monophosphates, whereas acid phosphatases do. Mutation close to the loop regions caused enough conformational changes making ApiTwo behave as an acid phosphatase without compromising its pyrophosphatase activity. The thermal denaturation experiments also indicated the same. The required orientation/conformation of disordered loop regions may not be retained on cooling after denaturation. The probability of attaining the right conformation on cooling is low. Therefore, pNPP and TPP hydrolysis are severely affected after heating and activity is not recovered after cooling. Whereas, the rigid helical regions are partially denatured on heating and the probability to renature is pretty high on cooling. Hence ATP and ADP hydrolysis are not significantly affected on heating the enzyme and the activity is recovered very well after cooling (Figure 10). These results clearly indicate the importance of the conformational changes induced in the loop due to the new mutations.

**Table 6. Affinity of substrates to different apyrases.**

| **Protein** | **Substrate** | **Activity** | **Affinity (Kₘ)** |
|---|---|---|---|
| ApiOne | ATP | Y | 20.0 µM |
| ApiTwo | | Y | 18.5 µM |
| EIEC apyrase | | Y | 21.0 µM |
| EIEC H116L | | Y | 24.0 µM |
| ApiOne | pNPP | N | - |
| ApiTwo | | Y | 0.76 mM |
| EIEC apyrase | | N | 25.0 mM |
| EIEC H116L | | Y | 3.5 mM |
| ApiOne | TPP | N | - |
| ApiTwo | | Y | 5.6 mM |
| EIEC apyrase | | Y | - |
| EIEC H116L | | Y | - |

### Materials and Methods for Examples 4-5

*Site directed mutagenesis done by PCR using specific primers.*

### PCR conditions

KOD polymerase (GENEX) was used for this PCR. The composition of the mix is as follows:

| **Component** | **Volume (µL)** |
|---|---|
| Water | 29 |
| Buffer (5X) | 10 |
| dNTPs (10 mM) | 1 |
| Forward primer (10 pmoles) | 1 |
| Reverse primer (10 pmoles) | 1 |
| Template DNA (50 ng) | 1 |
| Magnesium sulphate (50 mM) | 3 |
| DMSO (100%) | 3 |
| KOD enzyme | 1 |

As a control, a tube with all components mentioned were added except KOD polymerase.

### PCR conditions

| **Step** | **Temperature (°C)** | **Time (min)** |
|---|---|---|
| 1 | 98 | 5 |
| 2 | 98 | 0.5 |
| 3 | 48 | 1 |
| 4 | 68 | 7 |

Step 2-4 cycled for 18 times

To ensure that the template DNA (50 ng) is completely degraded since it will contain the wild type sequence, Dpnl (1 unit) was added to the remaining PCR mixtures and incubated for 1 hour at 37°C.

### Transformation of Mutant PCR mix into E. coli DH5α strains.

A fraction (20 µL) of the Dpnl digested PCR mix was transformed into *E.coli* DH5α by Calcium chloride method. Colonies were inoculated in LB media supplemented with 100 µg/mL ampicillin and incubated overnight at 37°C. Plasmids were extracted using standard procedures (Takara).

### Expression of recombinant ApiOne and ApiTwo mutants

ApiOne and ApiTwo mutant plasmids were transformed into *E.coli* RP codon plus by Calcium chloride method. Colonies were inoculated in LB media supplemented with 100 µg/mL ampicillin and 34 µg/mL chloramphenicol, and incubated overnight at 37°C. This culture was added to 500 mL of sterile LB medium with 100 µg/mL ampicillin and 100 µg/mL chloramphenicol such that the initial OD₆₀₀ₙₘ is around 0.08. The culture was grown at 37°C until OD₆₀₀ₙₘ reaches 0.5-0.6. Expression of ApiOne and ApiTwo mutants was induced by adding 1 mM IPTG and incubating the culture at 18°C for 20 hours. Culture not induced by IPTG was used as a control.

### Purification of ApiOne and ApiTwo mutants by affinity chromatography

Harvested cells of 500 mL culture (induced by IPTG) were resuspended in 30 mL binding buffer (50 mM Tris, pH 7.5, 150mM NaCl and 10 mM imidazole). The suspension was passed three times through a French press at pressure of 1000 bar.

Before binding to the resin, 1.0 mL of Ni- sepharose (GE Healthcare) resin was washed with 15 mL water. The beads were later washed with 15 mL binding buffer.

Lysates and the washed resin were mixed in 50 mL falcon tube and incubated for 1 hour at room temperature on a rocker.

After this, the lysate was drained out on a column and the beads were washed thoroughly with 200 mL binding buffer. The protein was then eluted in 1.0 mL fractions using the elution buffer (50 mM Tris, pH 7.5, 10% glycerol and 250 mM imidazole).

### pNPP assay

In a volume of 100 µL, 500 ng of pure ApiOne/ApiTwo/Double mutant proteins were incubated with 2.0 mM pNPP diluted in 50 mM Tri-HCl, pH 7.5 for 20 minutes at 37°C. The formation of product. Para-nitro phenol was monitored colorimetrically at 405 nm using multi-mode spectrophotometer (Biotek instruments).

### TPP assay

A stock of freshly prepared TPP (50 mM) was prepared before performing every assay. In a volume of 100 µL, 500 ng of pure ApiOne/ApiTwo/Double mutant proteins were incubated with 5.0 mM TPP diluted in 50 mM Tri-HCl, pH 7.5 for 20 minutes at 37°C. Hydrolysis of Thiamine pyrophosphate (TPP) releases Thiamine, which forms a thiochrome fluorophore on addition of hydrogen peroxide and horse radish peroxidase (HRP) in alkaline conditions **(NaOH- HRP-H₂O₂-** 1M NaOH, 5U/mL HRP, 50 mM H₂O₂ mixed in a volumetric ratio of 3:1:1). Fluorescence was measured using multi-mode spectrophotometer (Biotek instruments) with excitation at 368 nm and emission at 450 nm.

### ATP/ADP hydrolysis assay

In a volume of 100 µL, 500 ng of pure ApiOne or ApiTwo mutants were incubated with 1.0 mM ATP diluted in 50 mM Tri-HCl, pH 7.5 for 20 minutes at 37°C. To this 200 µL of Ammonium Molybdate Ferrous Ammonium Sulphate (AMFAS) reagent was added. AMFAS reagent was prepared by mixing equal volumes of reagent A and reagent B, where reagent A comprises of 5% (w/v) ammonium molybdate in 5 N H₂SO₄ and reagent B contains 1% (w/v) ferrous ammonium sulphate in double-distilled water. The amount of inorganic phosphate formed (product of ATP/ADP hydrolysis) is colorimetrically measured at 630 nm using multi-mode spectrophotometer (Biotek instruments).

### Affinity of recombinant proteins towards ATP, pNPP and TPP

In volume of 100 µL, 500 ng of pure ApiOne or ApiTwo mutants were incubated with different concentrations of substrate (ATP, pNPP and TPP). Respective colorimetric /fluorescence assays (mentioned above). The kinetic curves were plotted and the Michaelis Menten constant (Kₘ) was calculated using GraphPad Prism 5.0 software.

### Thermal stability of ApiOne and ApiTwo mutant proteins

ApiOne and ApiTwo (L66V) mutant proteins were incubated at different temperatures (25°C, 55°C and 70°C) for 2 h. Proteins stored at -20°C were used as control. The ability of these proteins to hydrolyse ATP/ADP (1.0mM), pNPP (2.0 mM) and TPP (5.0 mM) was studied by performing colorimetric/fluorescence assays described above. The ability of heated proteins to renature was also studied using these assays after cooling proteins to 25⁰C for 1 h. Percentage activity was calculated by considering activity of controls (proteins stored at -20°C) as 100%.

### Computational modelling of the structure of ApiOne

The primary sequence of ApiOne (SEQ ID NO:1) was submitted to the ITASSER server (https://zhanglab.ccmb.med.umich.edu/I-TASSER/) for the threading of the structure. The best template that was chosen by the server was the acid phosphatase (PDB entry: 1D2T) of *Escherichia blattae* (85% identity and 90% similarity). The structure was similar to the model reported by Babu et. al in 2002. The model was refined by loop refine tool in the Galaxy webserver (http://galaxy.seoklab.org/cgi-bin/submit.cgi?type=LOOP), ensuring that all residues fall in the desirable/allowed regions of the Ramachandran plot.

## Claims

1. An apyrase enzyme, **characterized by** that:
a. the apyrase comprises a polypeptide sequence having at least 90% sequence identity to the wild-type *Shigella flexneri* apyrase of SEQ ID NO:1,
wherein said sequence differs from SEQ ID NO:1 at least in that the sequence comprises at least one amino-acid substitution of a residue aligning with a residue selected from the group consisting of F53, L66 and E77;
and
b. the apyrase catalyzes the dephosphorylation of at least one organic phosphate with at least 10-fold lower Kₘ compared to the apyrase of SEQ ID NO:1.

2. The apyrase according to any of the preceding claims, wherein the apyrase catalyzes the dephosphorylation of the at least one organic phosphate with at least 50-fold lower, preferably at least 100-fold lower Kₘ compared to the apyrase of SEQ ID NO:1.

3. The apyrase according to any of the preceding claims, wherein the at least one organic phosphate is thiamine pyrophosphate (TPP) and/or para-Nitrophenylphosphate (pNPP).

4. The apyrase according to any of the preceding claims, wherein the apyrase exhibits a Kₘ of less than 30 mM for dephosphorylation of thiamine pyrophosphate (TPP), more preferably less than 20 mM, even more preferably less than 10 mM, most preferably less than 6 mM.

5. The apyrase according to claim 4, wherein the apyrase Kₘ for dephosphorylation of TPP is measured at pH7.5 and at a temperature of 37°C, preferably in a 50 mM Tris buffer at pH7.5.

6. The apyrase according to any of the preceding claims, wherein the apyrase exhibits a Kₘ of less than 2 mM for dephosphorylation of para-Nitrophenylphosphate (pNPP), more preferably less than 1 mM.

7. The apyrase according to claim 6, wherein the apyrase Kₘ for dephosphorylation of pNPP is measured at pH7.5 and at a temperature of 37°C, preferably in a 50 mM Tris buffer at pH7.5.

8. The apyrase according to any of the preceding claims, wherein the apyrase catalyses the dephosphorylation of ATP with a Kₘ differing less than 5-fold from that of the apyrase of SEQ ID NO:1, preferably less than 2-fold.

9. The apyrase according to any of the preceding claims, wherein the apyrase exhibits a Kₘ of less than 10 mM for the dephosphorylation of ATP, preferably less than 5 mM.

10. The apyrase according to any of claims 8-9, wherein the apyrase Kₘ for the dephosphorylation of ATP is measured at pH7.5 and at a temperature of 37°C, preferably in a 50 mM Tris buffer at pH7.5.

11. The apyrase according to any of the preceding claims, wherein the substitutions comprise the substitution F53V, the substitution L66V and/or the substitution E77V.

12. The apyrase according to any of the preceding claims, wherein the apyrase comprises a polypeptide sequence having at least 95% sequence identity to the wild-type *Shigella flexneri* apyrase of SEQ ID NO:1.

13. The apyrase according to any of the preceding claims, wherein the apyrase comprises a polypeptide sequence according to SEQ ID NO: 3 residues 1-246.

14. A use of an apyrase according to any of claims 1-13 for dephosphorylating organic phosphates.

15. A method for catalyzing the dephosphorylation of an organic phosphate, comprising contacting the organic phosphate with the apyrase of any of claims 1-13.

## Patentansprüche

1. Apyrase-Enzym, **dadurch gekennzeichnet, dass**:
a. die Apyrase eine Polypeptidsequenz umfasst, die mindestens 90 % Sequenzidentität mit der Wildtyp-Apyrase von*Shigella flexneri* aus SEQ ID NO:1 aufweist, wobei sich die Sequenz von SEQ ID NO:1 mindestens dadurch unterscheidet, dass die Sequenz mindestens eine Aminosäuresubstitution eines Rests umfasst, der mit einem Rest aus der Gruppe bestehend aus F53, L66 und E77 ausgerichtet ist;
und
b. die Apyrase die Dephosphorylierung von mindestens einem organischen Phosphat mit einem mindestens 10-fach niedrigeren Kₘ-Wert im Vergleich zu der Apyrase von SEQ ID NO:1 katalysiert.

2. Apyrase nach einem der vorstehenden Ansprüche, wobei die Apyrase die Dephosphorylierung des mindestens einen organischen Phosphats mit mindestens 50-fach niedrigerem, bevorzugt mindestens 100-fach niedrigerem Kₘ-Wert im Vergleich zu der Apyrase von SEQ ID NO:1 katalysiert.

3. Apyrase nach einem der vorstehenden Ansprüche, wobei das mindestens eine organische Phosphat Thiaminpyrophosphat (TPP) und/oder para-Nitrophenylphosphat (pNPP) ist.

4. Apyrase nach einem der vorstehenden Ansprüche, wobei die Apyrase einen Kₘ-Wert von weniger als 30 mM für die Dephosphorylierung von Thiaminpyrophosphat (TPP) aufweist, bevorzugt weniger als 20 mM, noch bevorzugter weniger als 10 mM, am meisten bevorzugt weniger als 6 mM.

5. Apyrase nach Anspruch 4, wobei der Apyrase-Kₘ-Wert für die Dephosphorylierung von TPP bei pH 7,5 und einer Temperatur von 37 °C gemessen wird, bevorzugt in einem 50 mM Tris-Puffer bei pH 7,5.

6. Apyrase nach einem der vorstehenden Ansprüche, wobei die Apyrase einen Kₘ-Wert von weniger als 2 mM für die Dephosphorylierung von para-Nitrophenylphosphat (pNPP) aufweist, bevorzugt weniger als 1 mM.

7. Apyrase nach Anspruch 6, wobei der Apyrase-Kₘ-Wert für die Dephosphorylierung von pNPP bei pH 7,5 und einer Temperatur von 37 °C gemessen wird, bevorzugt in einem 50 mM Tris-Puffer bei pH 7,5.

8. Apyrase nach einem der vorstehenden Ansprüche, wobei die Apyrase die Dephosphorylierung von ATP mit einem Kₘ-Wert katalysiert, der sich weniger als 5-fach von dem der Apyrase von SEQ ID NO:1 unterscheidet, bevorzugt weniger als 2-fach.

9. Apyrase nach einem der vorstehenden Ansprüche, wobei die Apyrase einen Kₘ-Wert von weniger als 10 mM für die Dephosphorylierung von ATP aufweist, bevorzugt weniger als 5 mM.

10. Apyrase nach einem der Ansprüche 8 bis 9, wobei der Apyrase-Kₘ-Wert für die Dephosphorylierung von ATP bei pH 7,5 und einer Temperatur von 37 °C gemessen wird, bevorzugt in einem 50 mM Tris-Puffer bei pH 7,5.

11. Apyrase nach einem der vorstehenden Ansprüche, wobei die Substitutionen die Substitution F53V, die Substitution L66V und/oder die Substitution E77V umfassen.

12. Apyrase nach einem der vorstehenden Ansprüche, wobei die Apyrase eine Polypeptidsequenz umfasst, die eine Sequenzidentität von mindestens 95 % mit der Wildtyp-Apyrase von *Shigella flexneri* aus SEQ ID NO:1 aufweist.

13. Apyrase nach einem der vorstehenden Ansprüche, wobei die Apyrase eine Polypeptidsequenz nach SEQ ID NO: 3, Reste 1-246, umfasst.

14. Verwendung einer Apyrase nach einem der Ansprüche 1 bis 13 zur Dephosphorylierung organischer Phosphate.

15. Verfahren zum Katalysieren der Dephosphorylierung eines organischen Phosphats, umfassend das Inkontaktbringen des organischen Phosphats mit der Apyrase nach einem der Ansprüche 1 bis 13.

## Revendications

1. Enzyme apyrase, **caractérisée en ce que** :
a. l'apyrase comprend une séquence polypeptidique présentant une identité de séquence d'au moins 90 % avec l'apyrase de type sauvage de *Shigella flexneri* de SEQ ID NO:1, dans laquelle ladite séquence diffère de SEQ ID NO:1 au moins **en ce que** la séquence comprend au moins une substitution d'acide aminé d'un résidu s'alignant sur un résidu choisi dans le groupe constitué par F53, L66 et E77 ;
et
b. l'apyrase catalyse la déphosphorylation d'au moins un phosphate organique avec un Kₘ au moins 10 fois inférieur à celui de l'apyrase de SEQ ID NO:1.

2. Apyrase selon l'une quelconque des revendications précédentes, dans laquelle l'apyrase catalyse la déphosphorylation de l'au moins un phosphate organique avec un Kₘ au moins 50 fois inférieur, de préférence au moins 100 fois inférieur à celui de l'apyrase de SEQ ID NO:1.

3. Apyrase selon l'une quelconque des revendications précédentes, dans laquelle l'au moins un phosphate organique est le thiamine pyrophosphate (TPP) et/ou le para-nitrophénylphosphate (pNPP).

4. Apyrase selon l'une quelconque des revendications précédentes, dans laquelle l'apyrase présente un Kₘ inférieur à 30 mM pour la déphosphorylation de la thiamine pyrophosphate (TPP), de préférence inférieur à 20 mM, de préférence encore inférieur à 10 mM, de préférence encore inférieur à 6 mM.

5. Apyrase selon la revendication 4, dans laquelle le Kₘ de l'apyrase pour la déphosphorylation de la TPP est mesuré à pH7,5 et à une température de 37°C, de préférence dans un tampon Tris 50 mM à pH7,5.

6. Apyrase selon l'une quelconque des revendications précédentes, dans laquelle l'apyrase présente un Kₘ inférieur à 2 mM pour la déphosphorylation du para-Nitrophénylphosphate (pNPP), de préférence inférieur à 1 mM.

7. Apyrase selon la revendication 6, dans laquelle le Kₘ de l'apyrase pour la déphosphorylation de la pNPP est mesuré à pH7,5 et à une température de 37°C, de préférence dans un tampon Tris 50 mM à pH7,5.

8. Apyrase selon l'une quelconque des revendications précédentes, dans laquelle l'apyrase catalyse la déphosphorylation de l'ATP avec un Kₘ différant moins de 5 fois de celui de l'apyrase de SEQ ID NO:1, de préférence moins de 2 fois.

9. Apyrase selon l'une quelconque des revendications précédentes, dans laquelle l'apyrase présente un Kₘ inférieur à 10 mM pour la déphosphorylation de l'ATP, de préférence inférieur à 5 mM.

10. Apyrase selon l'une quelconque des revendications 8 à 9, dans laquelle le Kₘ de l'apyrase pour la déphosphorylation de l'ATP est mesuré à pH7,5 et à une température de 37°C, de préférence dans un tampon Tris de 50 mM à pH7,5.

11. Apyrase selon l'une quelconque des revendications précédentes, dans laquelle les substitutions comprennent la substitution F53V, la substitution L66V et/ou la substitution E77V.

12. Apyrase selon l'une quelconque des revendications précédentes, dans laquelle l'apyrase comprend une séquence polypeptidique ayant au moins 95 % d'identité de séquence avec l'apyrase de type sauvage de *Shigella flexneri* de SEQ ID NO:1.

13. Apyrase selon l'une quelconque des revendications précédentes, dans laquelle l'apyrase comprend une séquence polypeptidique selon les résidus 1-246 de SEQ ID NO : 3.

14. Utilisation d'une apyrase selon l'une quelconque des revendications 1 à 13 pour déphosphoryler des phosphates organiques.

15. Procédé pour catalyser la déphosphorylation d'un phosphate organique, comprenant la mise en contact du phosphate organique avec l'apyrase selon l'une quelconque des revendications 1 à 13.
